# EUROPEAN PATENT APPLICATION

(11) **EP 4 000 625 A1**
(43) Date of publication of application: **25.05.2022**
(21) Application number: 20841437.5
(22) Date of filing: 13.07.2020
(51) Int. Cl.: A61K 35/32, A61K 38/39, A61K 31/726, A61K 31/711, A61K 9/00, A61P 19/02, A61L 27/36, A61L 27/20

(54) **COMPOSITION FOR REGENERATING GROWTH PLATE**

(30) Priority: 17.07.2019 KR 20190086412
(71) Applicant: Ajou University Industry-Academic Cooperation Foundation, Suwon-si, Gyeonggi-do 16499 (KR)
(72) Inventor: MIN, Byoung-Hyun, Anyang-si Gyeonggi-do 14101 (KR)
(74) Representative: Roos, Peter
(86) International application number: PCT/KR2020/009179
(87) International publication number: WO 2021/010702

(57) **Abstract**

The present invention provides a composition comprising fetal cartilage tissue-derived cells and a fetal cartilage tissue-derived extracellular matrix as active ingredients for regenerating a growth plate. The composition for regenerating a growth plate can inhibit bone bridge formation in a growth plate injury region without a scaffold and differentiate to a growth plate cartilage tissue to effectively fill and regenerate the injured region therewith, whereby the regenerated growth plate tissue can recover growth ability. In addition, the composition is compatible with and safe to biological tissues and is characterized by high reproducibility and homogeneity.

## Description

### TECHNICAL FIELD

The present disclosure relates to a composition for regenerating a growth plate.

### BACKGROUND ART

A growth plate is a cartilage tissue remaining between the middle and both ends of a limb bone, and is a part where length growth of the bone occurs. It is also called epiphyseal cartilage. As a fetal whose limb bones are all cartilage grows, the middle of the cartilage changes to bone and gradually spreads to both ends (primary ossification center). At both ends of the limb bones, a portion of the cartilage that has not yet been converted to bone is also converted into bone on its own (secondary ossification center). The remaining cartilage between the portions converted into bone becomes the growth plate, and around puberty, the growth plate also turns into bone and the length growth ends.

According to the Salter-Harris classification method, which classifies growth plate fractures into five types, Type 1 is the mildest fracture, often not observed on X-rays, and recovers within 3 weeks at the earliest and does not leave any other problems. Type 2 is a form in which a fracture is on the growth plate, an injured region and a non-injured region are formed in the growth plate, and surgical treatment may be performed if necessary. Types 3, 4, and 5 are more severe than Types 1 and 2. First, Type 3 is a form in which a fracture spreads to the frontal surface under the growth plate, which may present with partial growth disorders. Type 4 is a form in which a fracture passes through the growth plate and progresses to the joint, and requires surgical treatment. If left untreated, bone deformities may occur. Type 5 is a fracture caused by a vertical force, and among the growth plate fractures, may bring about the worst prognosis, such as growth disorders. Types 1 and 2 occur in 2-40% of cases, and Types 3 and 4 occur in 8-50% of cases.

In general, bone bridges are formed in 40-70% of injured growth plates, which cause problems in bone growth, resulting in serious problems such as length growth disorders and angular deformity. Currently, as a treatment method to solve these problems, deformity correction is performed by waiting until the growth plate is closed after occurrence of the disorders. However, it requires multiple surgeries and is accompanied by many sequelae such as residual deformity, nerve damage, and fillers. A surgical procedure that may be performed at the initial stage of deformation is removal of a bone formation site. After removing a site of ossification from the growth plate, an ossification shielding material such as autologous adipose tissue, silicone, or bone wax is inserted, but the ossification shielding material has a limitation in that it cannot perform the function of the growth plate. Therefore, there is a need for new therapeutic agents for fundamental treatment of the injury of the growth plate.

### DISCLOSURE OF THE INVENTION

### TECHNICAL GOALS

To solve the above issues, an aspect of the present invention provides a composition for regenerating a growth plate including a fetal cartilage tissue-derived cell and a fetal cartilage tissue-derived extracellular matrix as active ingredients.

Further, an aspect of the present invention provides a pharmaceutical composition for treating a growth plate injury including the composition for regenerating the growth plate.

### TECHNICAL SOLUTIONS

A composition for regenerating a growth plate according to an aspect of the present invention may include a fetal cartilage tissue-derived cell and a fetal cartilage tissue-derived extracellular matrix as active ingredients.

A pharmaceutical composition for treating a growth plate injury according to another aspect of the present invention may include the composition for regenerating the growth plate.

### ADVANTAGEOUS EFFECTS

A composition according to an embodiment of the present invention may be suitable for living tissues, have high reproducibility and homogeneity, and be differentiated into gel-type growth plate-like tissues by tissue engineering without an artificial scaffold.

The composition may inhibit bone bridge formation of a growth plate injury site and effectively fill and regenerate the injury site by differentiating into a growth plate cartilage tissue. Accordingly, a regenerated growth plate may recover functions such as growth ability.

In addition, the composition may be safe, do not require special surgical instruments during surgery, and be easily implanted regardless of size and shape of the injury site to treat the injured growth plate more conveniently.

### BRIEF DESCRIPTION OF DRAWINGS

FIG. 1 is a diagram schematically illustrating a growth plate treatment method using a growth plate gel according to an embodiment of the present invention.
FIG. 2 is a diagram illustrating a process of implanting a growth plate gel prepared according to Example 2 into an injured growth plate of a rabbit according to an experimental example of the present invention.
FIG. 3 shows micro-CT images of the rabbit growth plate injury model according to FIG. 2 and images showing tissue differentiation.
FIG. 4 shows immunostaining images of the rabbit growth plate injury model experiment according to FIG. 2.
FIG. 5 shows rat models according to an experimental example of the present invention.
FIG. 6 shows graphs illustrating measurement results of length and angular deformity of rat models according to an experimental example of the present invention.
FIG. 7 and FIG. 8 show images evincing tissue differentiation of rat models according to an experimental example of the present invention.
FIG. 9 shows safranin-O and u-CT images of 4, 14, 21, and 28 weeks of rat models according to an experimental example of the present invention.
FIG. 10 is a diagram illustrating observation of characteristics of growth plate gels according to culture methods.
FIG. 11 is a diagram illustrating characteristics of growth plate gels according to centrifuge conditions.
FIG. 12 shows a growth plate gel prepared according to an embodiment of the present invention.

### BEST MODE

Hereinafter, the present invention will be described in detail.

An embodiment of the present invention provides a composition for regenerating a growth plate including a fetal cartilage tissue-derived cell and a fetal cartilage tissue-derived extracellular matrix as active ingredients.

The term "fetal cartilage tissue-derived cell" as used herein is a generic term for cells separated from fetal cartilage tissue, and preferably, chondrocytes isolated after the cartilage tissue is completely digested using collagenase or the like.

The term "fetal cartilage tissue-derived extracellular matrix" as used herein refers to a biopolymer aggregate composed of molecules synthesized by cells from the fetal cartilage tissue-derived cells, and secreted and accumulated extracellularly, and may include fibrous proteins such as collagen and elastin, complex proteins such as glycosaminoglycan (GAG), and cell adhesion proteins such as fibronectin and laminin. The extracellular matrix forms the shape of a tissue with the proteins described above and determines physical properties of the tissue, and plays an important role in maintaining an environment of the cell.

The term "growth plate" as used herein refers to a part responsible for length growth of an animal and a part of the human body which makes the human grow taller, and is a kind of cartilage existing at an end of a long bone. Histologically, it belongs to hyaline cartilage, and is also called a bone end plate or an epiphyseal plate.

The term "regeneration" as used herein refers to actions of supplementing, restoring, repairing, and recovering a defect of a tissue or an organ occurring due to various causes to make it the same tissue or organ as before.

A composition for regenerating a growth plate according to an embodiment of the present invention includes water, collagen, GAG, and DNA, and may be a gel type. More specifically, it may include, based on 100 parts by weight of the total composition, 60 to 98 parts by weight of the water, 5 to 15 parts by weight of the collagen, 1 to 5 parts by weight of the GAG, and 0.5 to 3.5 parts by weight of the DNA.

The term "gel" as used herein refers to a jelly-like material and has physical properties ranging from soft and weak to strong and rough, and although the most of a gel is liquid, it acts like a solid as a whole due to its three-dimensional network structure. In Korean, " 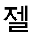 " and " 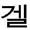 " may be used interchangeably to indicate the gel.

An embodiment of the present invention provides a pharmaceutical composition for treating a growth plate injury including the composition for regenerating the growth plate.

The term "growth plate injury" as used herein refers to injuries, damages, or defects occurring in the growth plate, the growth plate tissue, and the like, due to mechanical stimulation or an inflammatory response, and may also include diseases resulting therefrom.

The pharmaceutical composition according to the present disclosure may be inserted through a surgical incision during invasive point incision or trauma surgery to regenerate a growth plate injury or defect site. Bone bridge may be formed within 4 weeks after the growth plate is injured, and the pharmaceutical composition may remain for 3 to 5 weeks, preferably up to 4 weeks after being implanted into the growth plate to inhibit formation of the bone bridge, and then differentiate into growth plate-like tissues. In addition, after being regenerated into the growth plate-like tissue, the tissue may recover its function as the growth plate, and the growth ability may be continuously performed.

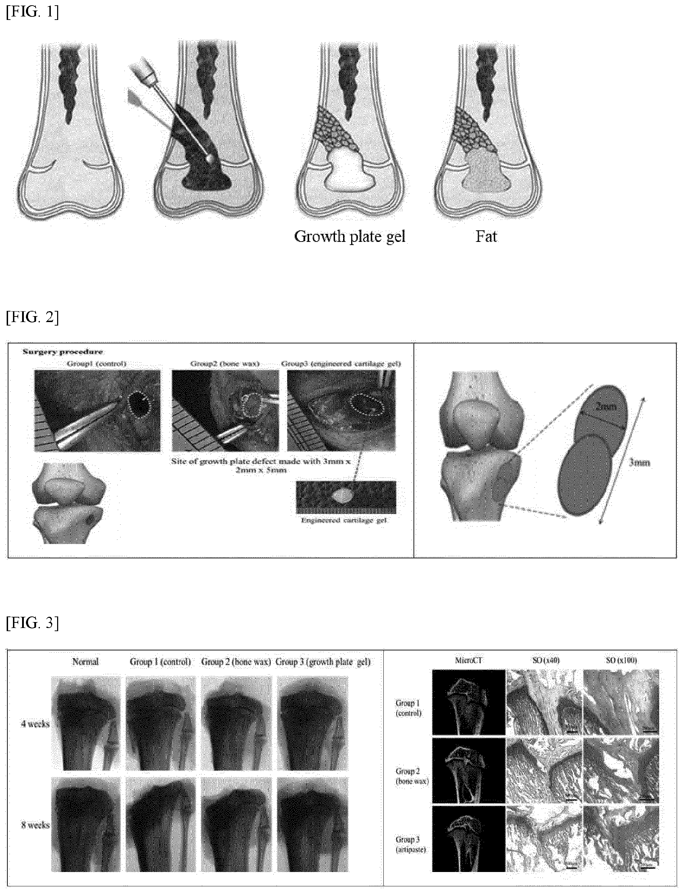

The pharmaceutical composition according to the present disclosure may be formulated in a form that can be directly injected into the growth plate injury or defect site, for example, may be a gel type including the cell and extracellular matrix with no scaffold. FIG. 1 is a diagram schematically illustrating a growth plate treatment method using a growth plate gel according to an embodiment of the present invention. Preferably, the composition may be prepared and implanted in the form of an injection including the gel, as shown in FIG. 1.

In addition, the injection may be prepared using an aqueous solution such as physiological saline, Ringel's solution, Hank's solution, or sterilized aqueous solution, vegetable oil such as olive oil, higher fatty acid ester such as ethyl oleic acid, and a non-aqueous solvent such as ethanol, benzyl alcohol, propylene glycol, polyethylene glycol, or glycerin, and may use any non-penetrating agent known in the art suitable for a barrier to be passed for mucosal penetration. It may further include a pharmaceutically acceptable carrier such as a stabilizer for prevention of deterioration such as ascorbic acid, sodium bisulfite, BHA, tocopherol, and EDTA, emulsifier, buffer for pH control, and preservative for preventing growth of microorganisms such as phenylmercuric nitrate, thimerosal, benzalkonium chloride, phenol, cresol, and benzyl alcohol. The injection may be stored for 24 hours in a refrigerated state of 3 to 5°C, preferably 4°C.

The pharmaceutical composition according to the present disclosure may be administered in a pharmaceutically effective amount. The "pharmaceutically effective amount" refers to an amount sufficient to treat the growth plate injury at a reasonable benefit/risk ratio applicable to medical treatment and not to cause side effects.

An effective dose level of the pharmaceutical composition may be determined differently according to factors including a purpose of use, age, sex, weight and health status of a patient, the type of disease, severity, activity of the drug, sensitivity to the drug, an administration method, administration time, an administration route and excretion rate, treatment duration, and drugs used in combination or at the same time, and other factors well known in the medical field. The pharmaceutical composition according to the present disclosure may be implanted at an appropriate dose depending on the size of the growth plate injury or defect site.

In addition, an embodiment of the present invention provides a method of preparing a composition for regenerating a growth plate.

The method for preparing a composition for regenerating a growth plate according to the present disclosure may include a first operation of culturing chondrocytes isolated from human fetal cartilage tissue, a second operation of obtaining a cell membrane including the cells and their extracellular matrix from the cultured chondrocytes, a third operation of obtaining a cell pellet by placing the obtained cell membrane in a cartilage differentiation medium and centrifuging it, and a fourth operation of culturing the obtained cell pellet in the cartilage differentiation medium to prepare a growth plate gel.

In the preparation method according to the present disclosure, the first operation is a operation of culturing the chondrocytes isolated from the human fetal cartilage tissue, and the chondrocytes may be obtained from cartilage tissue separated from a joint region of a fetal. According to an embodiment of the present invention, the cartilage tissue was isolated from a knee joint of a 12 to 15-week-old fetal.

The cartilage tissue may be treated with proteases such as collagenase or pepsin, and cells released accordingly are collected and centrifuged, and then precipitated chondrocytes may be seeded into a culture medium. The chondrocytes may be cultured in dulbecco's modified eagle medium (DMEM) supplemented with antibiotics, but is not limited thereto, and may be cultured in other commonly used culture media. According to an embodiment of the present invention, the chondrocytes were monolayer cultured in DMEM supplemented with 10% fetal bovine serum (FBS), 50 units/mL penicillin and 50 µg/mL streptomycin for 15 to 18 days.

In the method for preparing according to the present disclosure, the second operation is a operation of obtaining the cell membrane including the cells and their extracellular matrix from the chondrocytes cultured in the first operation. Unlike a general method for obtaining cells, in the present disclosure, it is possible to obtain the cell membrane including both the cells and extracellular matrix. According to an embodiment of the present invention, after culture, the medium was removed and 0.05% trypsin-EDTA was added to obtain the entire cell membrane including the cells and extracellular matrix at once without separating the cells into pipettes after treatment.

In the method for preparing according to the present disclosure, the third operation is a operation of putting the cell membrane obtained in the second operation into the cartilage differentiation medium and centrifuging to obtain the cell pellet, and the "cartilage differentiation medium" means a medium capable of supplying nutrients to support growth and survival in vitro and to be prepared as the composition for regenerating a growth plate, and may include all of the matrix secreted from the cells during culture period, and nutrients remaining after being consumed in the cell culture. According to an embodiment of the present invention, as the cartilage differentiation medium, dulbecco's modified eagle medium-high glucose (DMEM-HG) including 1% antibiotic-antimycotic, 1.0 mg/mL insulin, 0.55 mg/mL human transferrin, 0.5 mg/mL sodium selenite, 50 µg/mL ascorbic acid, 1.25 mg/mL bovine serum albumin (BSA), 100 nM dexamethasone, 40 µg/mL proline, and 10 ng/ml TGF-β was used.

The centrifugation of the third operation may be performed for 10 to 30 minutes at a rotation speed of 200 to 1500 × g. Preferably it may be carried out for 20 minutes at a rotation speed of 1000 × g. Although there is no change in a shape and physical properties of a tissue according to the centrifugation conditions, the most stable reproducibility may be illustrated at the rotation speed and execution time.

In the method for preparing according to the present disclosure, the fourth operation is the operation of preparing the growth plate gel by culturing the cell pellet obtained in the third operation in the cartilage differentiation medium, and characteristics of the growth plate gel may vary according to the culture method. According to an experimental example of the present disclosure, during high-density culture in a well plate, trends such as the longer the culture period, the thicker the membrane, the larger the overall tissue size and the softer the physical properties, appeared. In addition, as number of well increased, the membrane became thinner and smaller in size. Accordingly, the culture may be performed for 1 to 3 days in 6 to 24 well plate, and preferably, it may be cultured at a high density (HD) for 3 days in 6 well plate, at this time, tissue preparation reproducibility and preparation homogeneity may be higher. The growth plate gel prepared in this way may include only the cells and extracellular matrix without a scaffold, and more specifically, may include water, collagen, the GAG and DNA.

### MODES FOR CARRYING OUT INVENTION

Hereinafter, examples will be described in detail to help understanding of the present invention. However, the examples described below are merely illustrative of the present invention, and the scope of the present invention is not limited to the examples. The examples of the present invention are provided to more completely explain the present invention to a person skilled in the art.

### <Example 1> Isolation and culture of human fetal cartilage tissue-derived chondrocytes

For a preparation of a growth plate gel including fetal cartilage tissue-derived cells and a fetal cartilage tissue-derived extracellular matrix, chondrocytes were isolated from knee joint of 12-15 week-old fetal. After washing the cartilage tissue separated from the knee joint with phosphated buffered saline (PBS), it was cultured in dulbecco's modified eagle medium (hereinafter, DMEM, Gibco, Grand Island, NY) including 0.2% (w/v) collagenase (Worthington Biochemical Corp., Lakewood, NJ) in an incubator at 37° C, 5% CO₂ for 4 hours. After the cartilage tissue was completely digested, released chondrocytes were centrifuged at 1700 rpm for 10 minutes, precipitated chondrocytes were seeded to a tissue culture dish (150 mm (dia.) × 20 mm(h) at a density of 1×10⁶ cells per culture dish).

### <Example 2> Preparation of growth plate gel

The chondrocytes obtained in Example 1 were cultured as a monolayer for 15-18 days after dilution to 2×10⁵cells in dulbecco's modified eagle medium (DMEM) supplemented with 10% fetal bovine serum (FBS), 50 units/mL penicillin and 50 µg/mL streptomycin. After culture, the medium was removed and 0.05% trypsin-EDTA (Gibco) was added to obtain a cell membrane bound to extracellular matrix. To obtain the cell membrane bound to cells and the extracellular matrix, the entire cell membrane including the cells and extracellular matrix was obtained at once without separating the cells with a pipette after treatment with 0.05% trypsin-EDTA (Gibco).

The obtained cell membrane including the cells and extracellular matrix was put in a 50 ml tube including dulbecco's modified egle medium-high glucose (DMEM-HG) including 1% antibiotic-antimycotic, 1.0 mg/mL insulin, 0.55 mg/mL human transferrin, 0.5 mg/mL sodium selenite, 50 µg/mL ascorbic acid, 1.25 mg/mL bovine serum albumin (BSA), 100 nM dexamethasone, 40 µg/mL proline, 10 ng/ml TGF-β and centrifuged at 250 × g for 20 minutes to prepare a pellet-type structure.

The prepared cell pellet was placed in a culture dish including the same cartilage differentiation medium as the composition above, and cultured in an incubator at 37° C, 5% CO₂ for 1 week, 2 weeks, and 3 weeks to prepare a growth plate gel.

### <Experimental Example 1> Confirmation of tissue characteristics change and growth plate tissue activity according to growth plate gel injection in rabbit growth plate injury model

FIG. 2 is a diagram illustrating a process of implanting the growth plate gel prepared according to Example 2 into an injured growth plate of a rabbit according to an experimental example of the present invention. Since it is possible to confirm results of bone length and angular deformity, which are direct roles of the growth plate, in a rabbit, the rabbit is a model that may accurately confirm a role of the growth plate gel development targeted by treatment mechanism of the present disclosure.

Referring to FIG. 2, after piercing the growth plate part obliquely twice with 2 mm punch in the rabbit, after implanting the growth plate gel prepared in Example 2 of the present invention, an implant site was separated, and sectioned to thickness of 4 µm using a cryosectioning machine. A slide was prepared by sectioning, and bone growth form and movement of the implanted growth plate gel were checked. Group 1 is a negative control that has injured the growth plate, and group 2 is a positive control implanted with bone wax, which is currently most used in clinical practice.

FIG. 3 shows micro-CT images of the rabbit growth plate injury model according to FIG. 2 and images showing tissue differentiation.

Referring to FIG. 3, as a result of checking the micro-CT images of the rabbit growth plate injury model, at week 4, each group (normal (normal growth plate model), group 1 (growth plate injury model/negative control), group 2 (bone wax implant model/positive control), and group 3 (growth plate gel implant model)) did not exhibit a significant difference, but after 8 weeks, but there was a significant difference in each group. In the negative control group (group 1) and the positive control group (group 2), angular deformity of bone was occurring. The bone wax of the positive control group (group 2) had an effect of filling implant space, but there was a limit in maintaining implant material as bone tissue. On the other hand, the growth plate gel implant model (group 3) formed cartilage and was regenerated into normal cartilage having the same bone angle as that of the normal growth plate model (normal) so that the implanted site was not visible even when observed with the naked eye, thereby confirming the effectiveness of the growth plate gel.

In addition, through Safranin-O (SO), hematoxylin & eosin (H&E, HE) staining, it could be clearly confirmed that in the case of the negative control (group 1) and the positive control (group 2), bone tissue was formed at the injury site, whereas in the case of the growth plate gel implant model (group 3) cartilage tissue was located in a growth plate position even though it was thin.

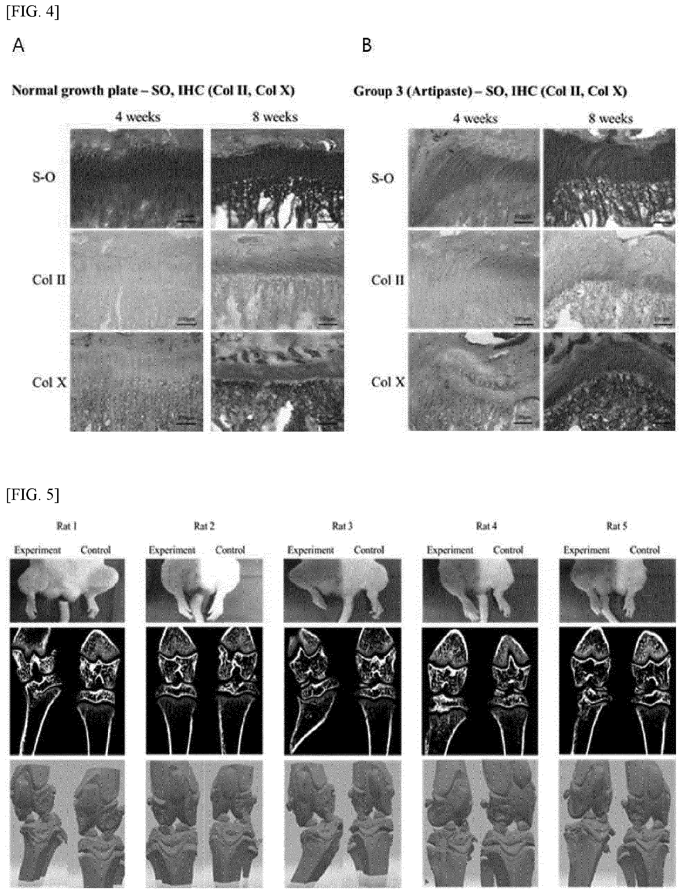

FIG. 4 shows immunostaining images of the rabbit growth plate injury model experiment according to FIG. 2. A shows immunostaining images of a rabbit normal growth plate model, and B shows immunostaining images of the growth plate gel implant model (group 3).

Referring to FIG. 4, it was confirmed that collagen and glycoprotein were formed in the growth plate gel implant model (group 3) similar to the case of the normal growth plate model (normal), and in the case of collagen X (col X), which is a bone formation stage, it was expected that ossification would be delayed or prevented as it was formed after 8 weeks.

### <Experimental Example 2> Confirmation of changes due to injection of growth plate gel in rat growth plate injury model

FIG. 5 shows rat models according to an experimental example of the present invention. In the case of the rabbit model according to the Experimental Example 1, the role of the growth plate may be confirmed as a clinical effect, but there is a limit to study a large number of animal tests and mechanisms of action, so growth plate injury and treatment effects were tested in the rat model.

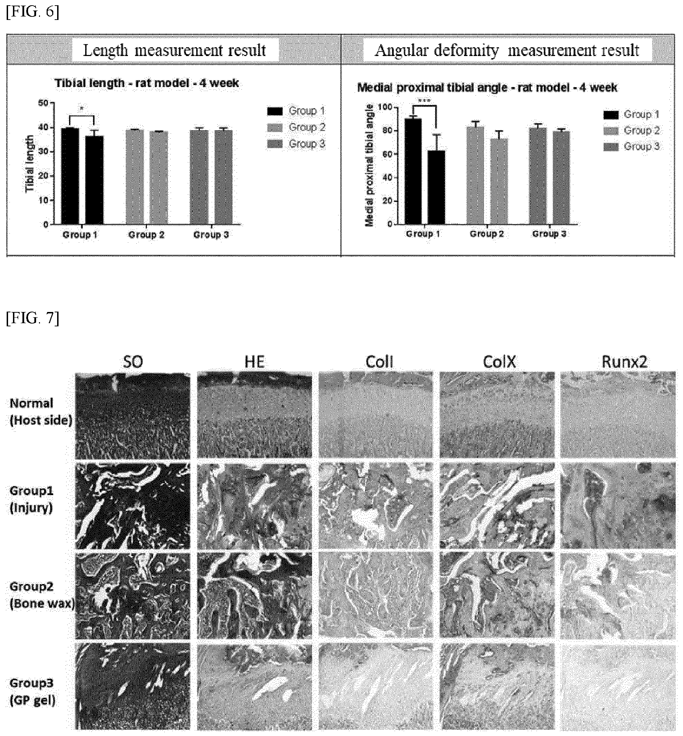

FIG. 6 shows graphs illustrating measurement results of length and angular deformity of rat models according to an experimental example of the present invention. The length growth and the angular deformity after 4 weeks of a rat growth plate injury model as a negative control (group 1), a bone wax implant model as a positive control (group 2) and a growth plate gel implant model (group 3) were measured.

Referring to FIG. 6, in the case of the length growth, a difference in the length growth was observed only in the rat growth plate injury model (group 1), and in the case of the angular deformity, the greatest angular deformity was observed in the rat growth plate injury model (group 1), and although not as much as in group 1, the angular deformity was also observed in the bone wax implant model (group 2).

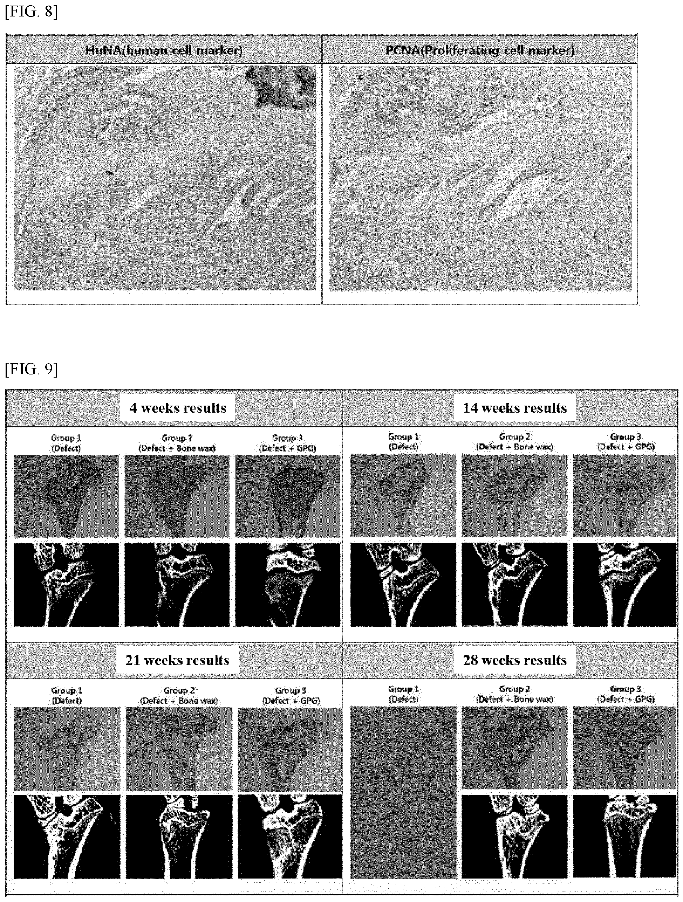

FIG. 7 and FIG. 8 show images evincing tissue differentiation of rat models according to an experimental example of the present invention.

Referring to FIG. 7 and FIG. 8, it was confirmed that as a result of safranin-O (SO) and hematoxylin & eosin staining, after 4 weeks, in the rat growth plate injury model (group 1) and the bone wax implant model (group 2), bone tissue was formed at the injury site, but in case of the growth plate gel implant model (group 3), bone differentiation did not occur and cartilage tissue was maintained. In addition, the length growth potential was confirmed by expression of a hypertrophic marker in a growing region.

In addition, in the growth plate gel implant model (group 3), implanted tissue was confirmed by staining a region where growth plate gel was expected to be implanted with humen cell marker (HuNA), and proliferating cells were confirmed through proliferating cell marker (PCNA)staining.

FIG. 9 shows safranin-O and u-CT images of 4, 14, 21, and 28 weeks of rat models according to an experimental example of the present invention.

Referring to FIG. 9, as a result of performing safranin-O and u-CT at 4, 14, 21, and 28 weeks after implant, it was confirmed that the same tendency appeared. In the rat growth plate injury model (group 1) and the bone wax implant model (group 2), no cartilage tissue was found at the growth plate injury site, and bone formation was confirmed by u-CT results. In particular, in the rat growth plate injury model (group 1), the angular deformity was observed at all weeks of age.

### <Experimental Example 3> Confirmation of characteristics of growth plate gel according to culture method

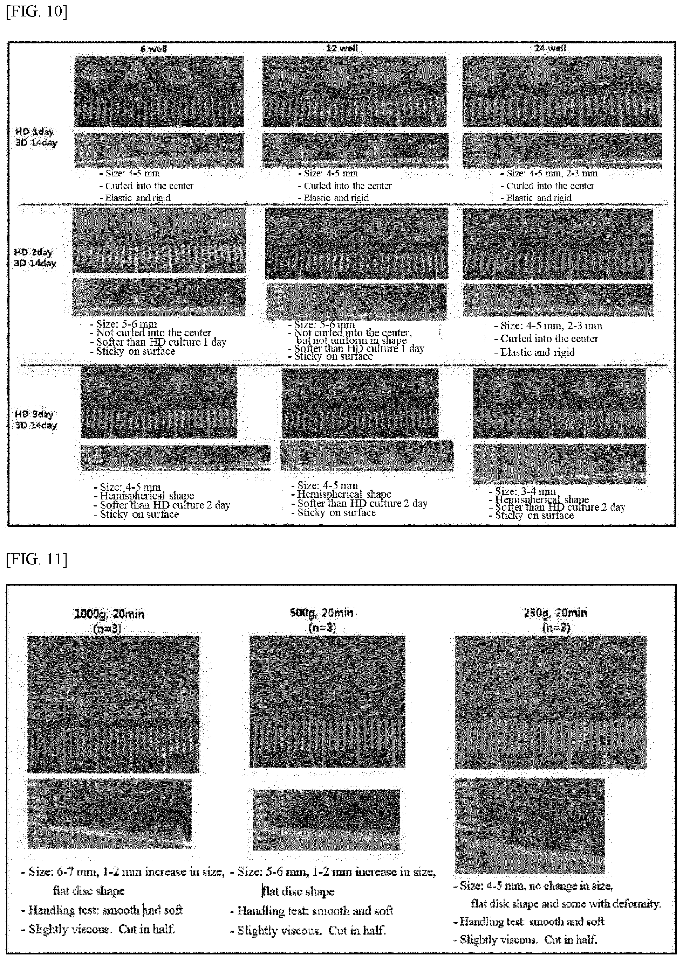

FIG. 10 is a diagram illustrating observation of characteristics of growth plate gels according to culture methods, and tissue size and physical properties (handling test) according to tissue culture vessels and high density (HD) culture period were checked.

Referring to FIG. 10, a method of culturing at high density (HD) in 6 well, 12 well, and 24 well for 1, 2, and 3 days was performed rather than a method of culturing for 5-6 days at HD in a previously setup 60 mm dish. When cultured at the HD in a culture plate, a membrane was well formed even on the first day, and as the period of the HD culture increased, the membrane became thicker, resulting in an increase in overall tissue size and softer physical property. In addition, the membranes tended to become thinner and smaller in size in the order of the 6 well, 12 well, and 24 well. Based on these results, the HD culture period of 3 days in the 6-well plate with high tissue preparation reproducibility and preparation homogeneity was established as an optimized process when preparing the growth plate gel.

FIG. 11 is a diagram illustrating characteristics of growth plate gels according to centrifuge conditions, and when conditions of the centrifugation, which is an operation prior to moving to a three-dimensional culture after a two-dimensional culture, were adjusted, it was check how they affected the physical properties (handling test) of the growth plate gel.

Referring to FIG. 11, as a result of setting the previously established centrifugation conditions of 1000 × g, 20 minutes, 500 × g, 20 minutes, and 250 × g, 20 minutes, there was no change in the shape and physical properties of the tissues according to the centrifugation conditions and the tissue size showed a tendency to decrease from 1000 × g to 250 × g. Based on these results, the tissue size was the largest at the condition of 250 × g, 20 minutes, but in a drying process of the high-density cultured membrane, the most stable reproducibility was shown in the case of 1000 × g and 20 minutes, so 1000 × g, 20 minutes was established as the optimized process.

### <Preparation Example 1> Growth plate gel

FIG. 12 shows a growth plate gel prepared according to an embodiment of the present invention.

Referring to FIG. 12, the growth plate gel is a colorless circular (sphere) artificial cartilage gel, and it was confirmed to include 20±15 µg/mg (or 2±1.5%) of DNA, 800±180 µg/mg (or 80±18%) ) of water, 30±20 µg/mg (or 3±2%) of glycosaminoglycan (GAG), and 100±50 µg/mg (or 10±5%) of collagen.

In addition, when a finished drug product was made with chondrocytes of 4×10⁶ cells or more, weight was measured to be 50±20 mg, and the physical properties of the finished drug product of gel-type artificial cartilage were measured to be 10±8 kPa.

### <Comparative Example 1> Comparison of bone wax and silastic

When comparing existing products, bone wax and silastic (silicone), and a growth plate gel according to an example of the present invention, the existing product are dense gel with excellent bone shielding function but materials with a simple shielding function and have an issue of generating growth plate defect sites.

On the other hand, the growth plate gel has an excellent shielding function as a tissue engineering preparation, tissue restoration is possible through cell differentiation and extracellular matrix regeneration, and further remodeling and growth are possible.

Although specific parts of the present invention have been described in detail, it is clear for a person skilled in the art that these specific descriptions are merely preferred example embodiments and the scope of the present invention is not limited thereto. In other words, the substantial scope of the present invention is defined by the appended claims and their equivalents.

## Claims

1. A composition regenerating a growth plate comprising a fetal cartilage tissue-derived cell and a fetal cartilage tissue-derived extracellular matrix as active ingredients.

2. The composition of claim 1, wherein the composition comprises water, collagen, glycosaminoglycan (GAG), and DNA, and is a gel type.

3. The composition of claim 2, wherein the composition comprises, based on 100 parts by weight of the total composition, 60 to 98 parts by weight of the water, 5 to 15 parts by weight of the collagen, 1 to 5 parts by weight of the GAG, and 0.5 to 3.5 parts by weight of the DNA.

4. A pharmaceutical composition for treating a growth plate injury comprising the composition of any one of claims 1 to 3.

5. The pharmaceutical composition of claim 4, wherein the composition is inserted through a surgical incision during invasive point incision or trauma surgery to regenerate a growth plate injury or defect site.

6. The pharmaceutical composition of claim 4, wherein the composition remains for 3 to 5 weeks after being implanted into a growth plate to inhibit bone bridge formation, and then differentiates into tissue of the growth plate.

7. The pharmaceutical composition of claim 4, wherein the composition is prepared in the form of an injection and is capable of being stored for 24 hours in a refrigerated state at 3 to 5°C.
